**Europäisches Patentamt**

(19) **European Patent Office**

**Office européen des brevets**

(11) Publication number: **0 037 416**

**B1**

(12) **EUROPEAN PATENT SPECIFICATION**

(45) Date of publication of patent specification: **16.05.84**

(51) Int. Cl.³: **C 12 Q 1/16**

(21) Application number: **80902099.3**

(22) Date of filing: **30.09.80**

(86) International application number:
**PCT/US80/01313**

(87) International publication number:
**WO 81/00858 02.04.81 Gazette 81/08**

(54) **MICROBIAL MONITOR.**

(30) Priority: **01.10.79 US 80327**

(43) Date of publication of application:
**14.10.81 Bulletin 81/41**

(45) Publication of the grant of the patent:
**16.05.84 Bulletin 84/20**

(84) Designated Contracting States:
**DE FR GB NL SE**

(56) References cited:
**US-A-3 129 144**
**US-A-3 817 239**
**US-A-3 844 894**
**US-A-3 935 073**
**US-A-3 952 729**
**US-A-3 956 070**
**US-A-3 997 404**
**US-A-4 021 308**
**US-A-4 057 470**
**US-A-4 071 315**

(73) Proprietor: **EIKMAN, Edward A.**
**5116 Longfellow Avenue**
**Tampa, FL 33609 (US)**

(72) Inventor: **EIKMAN, Edward A.**
**5116 Longfellow Avenue**
**Tampa, FL 33609 (US)**

(74) Representative: **Reichel, Wolfgang, Dipl.-Ing.**
**et al**
**Reichel und Reichel Parkstrasse 13**
**D-6000 Frankfurt am Main 1 (DE)**

(56) References cited:
**APPLIED MICROBIOLOGY, vol. 22, no. 5,**
**November 1971, US H.J. DE BLANC Jr. et al.**
**"Automated radiometric detection of bacteria**
**in 2,967 blood cultures", pages 846-849**
**JOURNAL OF LABORATORY AND CLINICAL**
**MEDICINE, vol. 75, no. 3, March 1970, F. DE**
**LAND et al. "Automated radiometric detection**
**of bacterial growth in bloodcultures", pages**
**529-534**

Courier Press, Leamington Spa, England.

## Description

This invention relates to a system for monitoring fluids for a microbial contamination and, more particularly, to such a system designed to automatically sample potentially contaminatable fluid and indicate when contamination of such fluid occurs.

The invention is particularly useful for detecting contamination of urine in catheterized patients, who are susceptible to infection of the urinary tract resulting in contamination of the urine.

Prior to the present invention, contamination of urine in catherized patients was monitored by periodically taking individual urine samples and culturing each sample to determine the presence of contamination. An early detection of microbial growth was made by using a growth medium containing substances capable of emitting radioactive gases which were analysed in a radioactive gas detector, such as an ionization chamber to rapidly detect the presence of radioactivity. In this connection reference is made, for example, to U.S. Patent No. 3,935,073 and Journal of Laboratory and Clinical Medicine, Vol. 75, No. 3, March 1970, F. De Land et al. "Automated radiometric detection of bacterial growth in bloodcultures", pages 529 to 534.

However, in the prior art the sample, such as the urine sample, had to be sent to a laboratory to be cultured so that a substantial amount of time would normally elapse between the time that the urine sample was taken and a determination made that the urine was contaminated. As a result, by the time it had been determined that the urine sample was contaminated, the patient often already had a massive infection.

The present invention overcomes this problem of the prior art technique by providing a much earlier indication of the presence of contamination the fluid from which the samples are taken. This is achieved by providing at the site of a patient a liquid culture medium and periodic urine samples are automatically fed into the same tracer-labeled liquid culture medium. When contamination of the urine occurs, a contaminated sample will be fed into the culture medium and be cultured. As a result, the growth medium will evolve gas, which will be detected by a detector to provide an early indication of the presence of such gas thus indicating the presence of microbial contamination in the urine. The system differs from the prior art systems, not only in the automatic sampling of the urine from the patient, but also in the fact that each urine sample is fed into the same culture medium as the previous samples. Each urine sample can be fed into the same culture medium because the system is designed only to detect the presence of contamination and the samples of urine taken from the patient prior to infection of the patient would be sterile and would not affect the ability of the culture medium to respond to a later contaminated sample.

It will be evident that the system as described is also applicable to detect the presence of contamination in other fluids by automatically taking periodic samples of the fluids including fluids from wound drainage, dialysis fluids, peritoneal cavity drainage, cerebro-spinal fluid drainage, exhaled air, fluids undergoing or intended for intravenous administration or inhalation, fluids in industrial processes including food preparations, particles of solids or liquids within fluid streams, or any normally sterile or culturable fluid.

Brief Description of the Drawings

Fig. 1 is a schematic diagram illustrating the system of the present invention;

Fig. 2 is a schematic illustration of an embodiment of the radioactive gas detector of the scintillation type for use in the system of Fig. 1;

Fig. 3 is a schematic illustration of another embodiment of the radioactive gas detector employing an ionization chamber;

Fig. 4 is a schematic illustration of yet another embodiment of the radioactive gas detector employing a Geiger-Muller counter;

Fig. 5 is a schematic illustration of another embodiment of the radioactive gas detector employing a semiconductor radioactivity detector;

Fig. 6 is a schematic illustration of another embodiment of the system using a nuclear magnetic reasonance analyzer to detect change in the nuclide contents of the gas mixture composition due to the evolution of metabolic products;

Figs. 7 and 8 schematically illustrate the two operable positions of one embodiment of the sampler of the system in Fig. 1;

Figs. 9 and 10 schematically illustrate the two operative positions of another embodiment of the sampler of Fig. 1;

Fig. 11—16 are schematic illustrations of another embodiment of the sampler in Fig. 1, with provision for continuous sampling and concentration of the sample; and

Figs. 17—19 are schematic illustrations of another embodiment of the sampler in Fig. 1, with provision for continuous sampling and concentration of the sample.

Description of the Preferred Embodiments

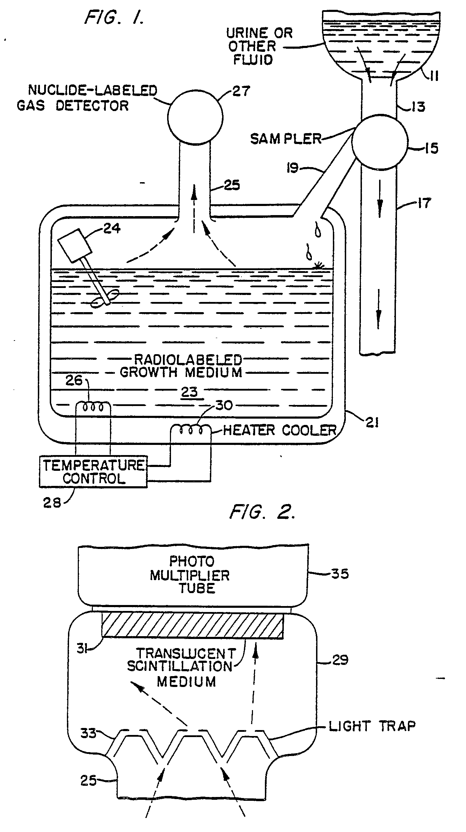

As shown in Fig. 1, the liquid, such as urine, being monitored from a source 11 flows through tubing 13 to an intermittent sampler 15. Fluid normally exits from the intermittent sampler 15 through tubing 17 to a suitable collector or waste disposal system, not shown. Periodically, at regular intervals, the sampler 15 directs a small sample from the fluid automatically through tubing 19 into a container 21, which contains a suitable tracer-labeled liquid growth or culture medium 23. For catheterized patients, the sampling rate of urine samples should be in the range of once every ten minutes to once every hour. For industrial applications, the sampling rate may be once every six hours, or very small samples may be taken at more frequent intervals.

The container 21 has an insulating jacket and is provided with a suitable agitator 24 to stir the growth medium. A thermostat 26 senses the temperature within the growth medium 23 and signals a temperature control 28, which operates a heater-cooler 30 to maintain the temperature within the medium 23 to promote the growth of micro-organisms. This temperature will usually be selected to be 37°C.

The growth medium 23 contains one or more tracer-labeled nutritional substrates, which produce tracer-containing gaseous products when metabolized. The composition of the medium is selected to favor the growth of categories or types of organisms of greatest importance or interest and inhibit the growth of other types of organisms. The composition of the medium allows evolution of the appropriate gaseous products rather than retention of the products in solution. For example, the pH of the solution may be selected to release carbon dioxide. The culture medium is of sufficiently large volume so that the successive samples introduced into the medium through the tubing 19 have no substantial effect on the characteristics of the growth medium over a substantial period of time and for a substantial number of samples.

The space in the container 21 over the growth medium 23 communicates through a conduit 25 with a gas detector 27. When the growth medium 23 evolves tracer containing gas, some of this gas will pass through the conduit 25 into the detector 27 and be detected thereby thus indicating the presence of micro-organisms in the sample introduced into the growth medium and, accordingly, indicating contamination of the fluid being monitored.

Fig. 2 schematically illustrates an example of a radioactive gas detector which may be used in the system of Fig. 1. Nutritional substrates in growth medium 23 include radionuclides emitting radioactivity of sufficiently low energy that little radioactivity reaches the radioactivity detector unless a radioactive gas is produced by metabolism of the nutritional substrates. As shown in Fig. 2, the detector comprises a chamber 29 having a translucent scintillation medium 31, such as an activated sodium iodide crystal radiation detector contained in a thin, gas-tight envelope, positioned at the top thereof.

A preferred embodiment uses as scintillation medium 31 a translucent substrate using an organic scintillation medium such as "Liqui-fluor", commerically available from the New Englnd Nuclear Company. In the growth medium, the substrate is moistened with an aqueous alkaline solution, 0.1 N KOH, in order to favor the dissolution of gaseous Carbon-14 dioxide, thereby bringing the radioactive gas in close proximity to the scintillation medium 31. A light trap 33 is provided across the mouth of the chamber 29 which communicates with the conduit 25, the light trap permitting gas to flow into the chamber 29. When the radio emission from the radioactive gases impinges upon the scintillation medium 31, it will emit light scintillations, which, in turn, will be detected by a photomultiplier tube 35. The light trap 33 prevents ambient light from causing false indications by the photomultiplier tube 35, whenever light is admitted, for example, in the process of renewing the growth medium 23.

In this embodiment of the system, the photomultiplier tube is protected from ambient or fluorescent light from sources other than scintillations induced by radioactive gases in the chamber 29. This protection is accomplished by the use of opaque construction materials and coatings to exclude ambient light from the system.

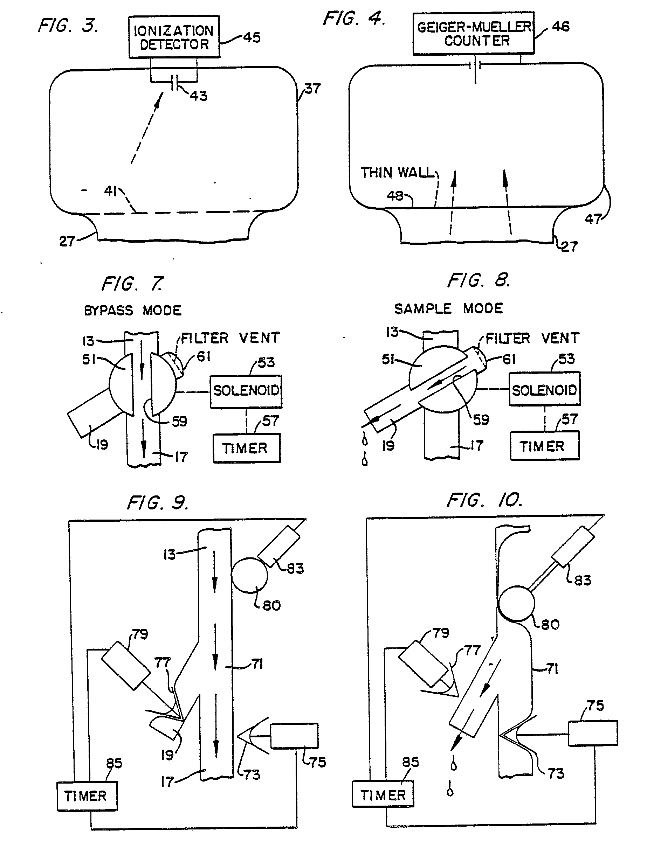

Alternatively, the radioactive gas detector, as shown in Fig. 3, may comprise an ionization chamber 37, which is separated from the conduit 27 by a semipermeable membrane 41. The ionization chamber 37 contains electrodes 43 of an ionization detector 45. When radioactive gas passes through the membrane 41 and into the chamber 37, it will cause ionization of the gases in the chamber 37. This ionization will be detected by the ionization detector 45, which will then provide an indication that the fluid being monitored has become contaminated. The purpose of the membrane 41 is to exclude particles from the ionization chamber 37.

A third embodiment of the radio gas detector is illustrated in Fig. 4 which employs a Geiger-Muller counter to detect the presence of radio-active gases in a chamber 47. In this embodiment, the chamber 47 communicates with the conduit 27 through a thin wall 48. This wall is sufficiently thin to admit emissions from the radioactive gas in conduit 25.

A fourth embodiment is illustrated in Fig. 5, in which a semiconductor radioactivity detector 49 is used to detect the presence of radioactive gases in chamber 47.

Another embodiment of the invention is illustrated in Fig. 6, in which a nuclear magnetic resonance analyzer 50 is used to detect change in the nuclide contents of the gas mixture in chamber 47. This embodiment is particularly useful for the detection of the evolution of gases containing stable nuclides as evidence of contamination in the liquid growth medium. In this embodiment, the application of the invention is realized without the use of radioactive nuclides that may result in hazards associated with their use or disposal. For example, gaseous Carbon-13 dioxide may be detected as a product of the metabolism by contaminating micro-organisms.

The gas detection apparatus used in any of these embodiments is provided with an audible alarm, now shown, or other signaling device in order to alert intertested personnel to the presence of signals of contamination when detected.

As shown in Figs. 7 and 8, the intermittent sampler 15 may comprise a rotary valve 51 operated by a solenoid 53 controlled in turn by a timer 57. The rotary valve 51 comprises passageway 59 which, in the position shown in Fig. 7, directs the fluid from the tube 13 to the tube 17. By means of the solenoid 53, the valve 51 can be moved to the position shown in Fig. 8 in which it connects between the tube 19 and a vent 61 closed by a filter. At regular intervals, the timer 57 will energize the solenoid 53, which in turn will actuate the valve 51 to the position shown in Fig. 7. When the valve is actuated to the position shown in Fig. 8, the sample of fluid contained in the conduit 59 will be directed into the chamber 21 through the tube 19.

In the embodiment of intermittent sampler 15 shown in Figs. 9 and 10, the tube 19 and the tubes 13 and 15 are implemented by flexible tubing with the tube 19 and 17 joining the tube 13 in the Y-connection 71. A stop clamp 73 operated by a solenoid 75 is provided to selectively close the tubing 17 just below the Y-connection 71. A stop clamp 77 operated by a solenoid 79 is provided to selectively close the tubing 19 just below the Y-connection 71. A roller 80 operated by a solenoid 83 is provided to selectively close and flush the tubing 13 above the Y-connector 17 as shown in Fig. 10. The solenoids 75, 77 and 83 are controlled by a timer 85. In their normal positions, the solenoids will be de-energized in which case the clamp 77 will close the tube 19, the clamp 73 will leave the tube 17 open and the clamp 80 will leave the tube 13 open as illustrated in Fig. 9. At periodic intervals, the timer energizes the solenoids 75, 77, and 83 to cause the clamp 73 to momentarily close the tube 17, the clamp 77 to momentarily open the tube 19, and the roller 80 to momentarily close the tube 13 so that a small sample of fluid of limited volume may flow through the tube 19 into the chamber 21. When the solenoid 83 is energized to actuate the roller 80, the roller 80 not only closes the tube 13, but also, as shown in Fig. 10, moves along a short portion of the tube 13 toward the Y-connector 17 to flush fluid in the tube 13 through the tube 19 into the chamber 21. The device represented by the roller 80 and the flexible tube 13 is referred to as a peristaltic pump. A moment after the flushing of the fluid by roller 80, the solenoids 75, 77 and 83 are de-energized and the clamps 73 and 75 return to the position shown in Fig. 9 and the roller 80 returns to the position shown in Fig. 9.

It will be observed that with both the embodiments of the intermittent sampler, the sample that is intermittently directed into the chamber 21 is controlled in volume so that only a few drops may flow into the chamber 21 at the time of taking of each sample. With the embodiments of Figs. 9 and 10, it is limited to the amount contained above the clamp 77 and below the clamp 80. The size of the periodic sample is limited to be a small amount in this way so as not to have a significant effect upon the desirable characteristics of the growth medium. Preferably, the volume of each sample is about 0.1 milliliters and should not exceed 1 milliliter. The growth medium preferably has a volume on the order of 100 times the volume of the samples and at a minimum, should be at least 10 times the volume of the samples.

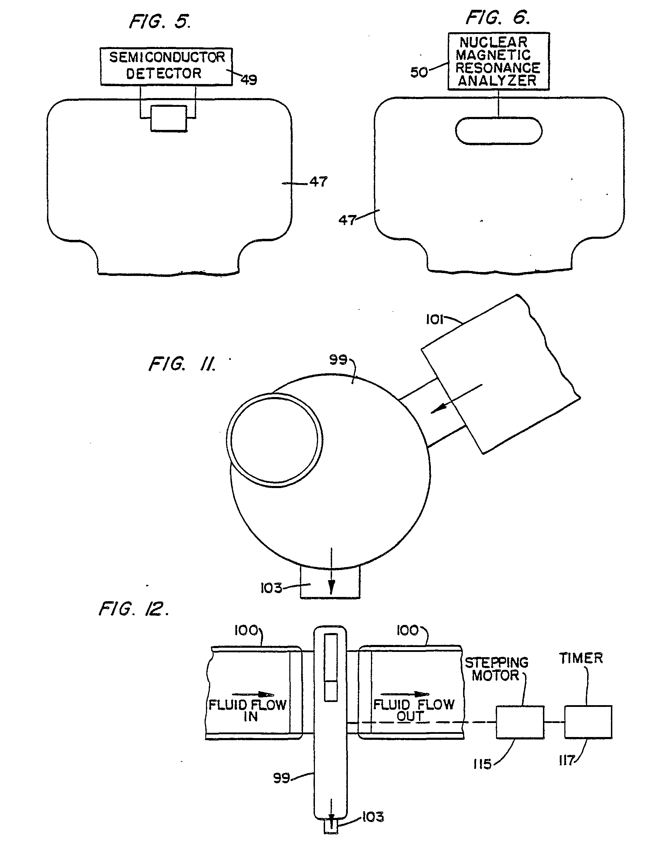

Whenever contamination in the fluid is intermittent or in low concentration, it is necessary that sampler concentrate material from the fluid flow to be monitored. Figs. 11—19 illustrate two preferred embodiments of the sampler that concentrates material from the fluid flow. In these embodiments, components from the fluid stream that are suspected to be associated with contamination are collected nearly continuously in order to increase the likelihood of early detection of contamination. The collected components are then introduced into the growth medium at selected intervals.

In the embodiments illustrated in Figs. 11 through 15, a sampler 99 is provided to successively and periodically transport porous collection discs into a stream being monitored and then deliver the discs to the growth medium. Fig. 11 schematically illustrates the apparatus viewed in elevation in the direction of fluid being monitored to the sampler 99 and Fig. 12 illustrates the apparatus viewed perpendicular to the direction of fluid flow. As shown in Fig. 11, the sampler 99 is provided with suitable means 101 of supplying fresh discs for collecting samples to the sampler 99 and with a suitable passageway 103 to deliver discs to the chamber containing the liquid growth medium. As shown in Fig. 11, the sampler 99 is connected in tubing 100 containing the flowing fluid that is to be monitored. In Fig. 11, the apparatus is illustrated without the tubing 100 connected.

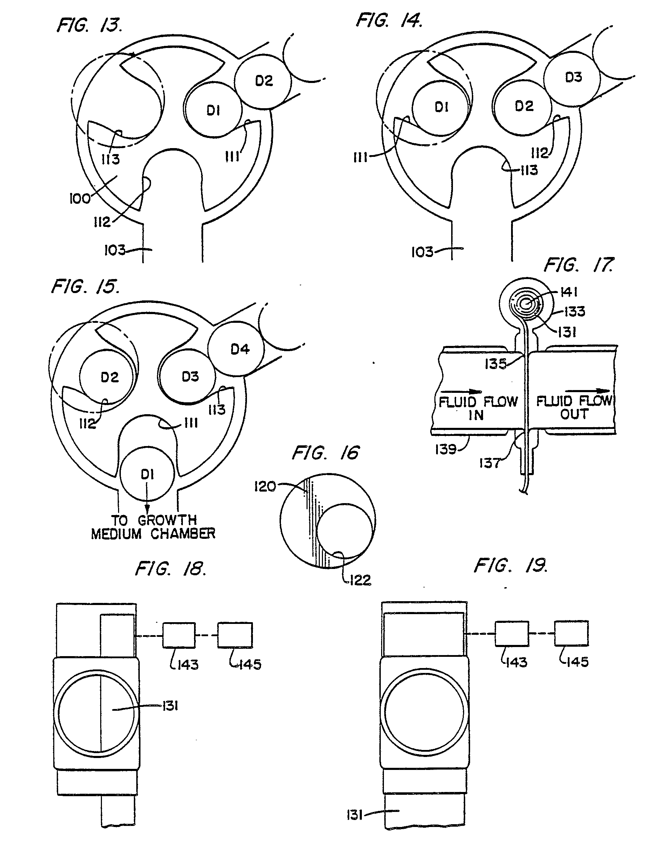

Figs. 13, 14 and 15 illustrate a cutaway view of the sampler 99 and, as shown in these figures, the sampler 99 contains a turntable 105, in the edges of which are defined three disc receiving pockets 111—113. Figs. 13, 14 and 15 illustrate the turntable 105 in the sampler 99 in each of three rest positions. In the rest position shown in Fig. 13, a collection disc D1 from the supply means 105 calls into the pocket 111 in the turntable 105. The turntable 105 is rotated counterclockwise in 120° increments at predetermined intervals by means of a stepping motor 115 controlled by a timer 117 as shown in Fig. 12. The sequence of rotation of the turntable is from the rest position of Fig. 15. In the rest position of Fig. 14, the disc D1 is introduced into the fluid stream flowing from the tubing 100 to the sampler 99 and a fresh disc is allowed to enter pocket 112 in the turntable 105.

The turntable rotates after a specified collection interval to the position shown in Fig. 15. This brings fresh disc D2 into collection position, drops the disc D1 that previously occupied the collection position into the growth medium 23 for culture, and allows another fresh disc D3 to enter pocket 113 in the turntable.

Each time a disc is brought into the collection position, fluid from the tubing 100 comes in contact with the disc when it is positioned in the flow of the fluid and is allowed to flow around it so that the disc collects material from the fluid flow by adsorption and/or by filtration. Alternatively, as shown in Fig. 16, a baffle 120 may be provided in the sampler 99 in the flow path of the fluid with the baffle containing an aperture 122 aligned with and corresponding in size to the size of the discs so that the baffle directs substantially all of the fluid to flow through a disc when it is positioned in the collection in the fluid stream. In this embodiment, the disc collects material from the fluid flow by filtration.

The turntable continues to rotate in 120° increments at specified intervals, adding a collection disc to the incubation medium with each incremental rotation. The discs are sufficiently small in relation to the volume of the incubation medium so that the circulation of the medium remains sufficient for growth purposes after a large number of discs have been added. In some cases, the disc may be so constructed that after immersion in the growth medium for a period of time equal to several of the specified sampling intervals, the disc disintegrates into many particles when the growth medium is agitated. This subdivision of the disc reduces any tendency of the discs to prevent free circulation of growth medium.

In the embodiments of the invention shown in Figs. 17—19, a coil of fresh sample strip 131 is wound in a container 133. Fig. 17 schematically illustrates the apparatus in deviation viewed perpendicularly to the direction of flow of the fluid being monitored. Figs. 18 and 19 each illustrate an alternative arrangement of the apparatus viewed in the direction of the flow of fluid. As shown in Fig. 17, the end of the sample strip is threaded through an entrance slot 135 and an exit slot 137 in the sidewall of tubing 139 so that the sample strip passes through the interior of the tubing 139 to come into contact with the fluid flowing through the tubing 139. The sample strip is wound on an axle 141 which is driven by a stepping motor 143 under the control of a timer 145 (see Figs. 18 and 19). The stepping motor 143 incrementally advances the sample strip to the tubing 139 and the sample strip collects material from the fluid flow by absorption and filtration. From the exit slit 137, the strip with the collected samples is fed to the growth medium where the sample is incubated and contamination detected as described above.

Instead of incrementally advancing of the sample strip by driving the axle on which the sample strip is wound, the strip may be advanced incrementally by pinch rollers pulling the strip through the exit slot 37.

As shown in Fig. 18, the strip may just occupy part of the tubing allowing for part of the fluid to bypass the sample strip or the strip may be arranged to completely fill the cross-section of the tubing, as shown in Fig. 19, so that all of the fluid flows through the sample strip.

In the embodiments described above, when sufficient uncontaminated samples have been introduced into the growth medium that they would begin to have a significant effect on the grown medium, the growth medium is replaced with a fresh growth medium. For this reason, the chamber containing the growth medium is made disposable, and a fresh growth medium is provided simply by replacing the chamber 21 with a new chamber containing the fresh medium. Preferably the period at which the growth medium is replaced is selected to be short enough that the total volume of the samples introduced into the growth medium does not exceed 25 percent of the volume of the growth medium. The growth medium would normally be replaced once every day or two. Samples are normally added to a single growth medium for culture.

More than one growth medium is used whenever more than a single variety of microorganism is suspected as a possible contaminant, and the growth requirements of the two or more suspected varieties of contaminant differ so that a growth medium satisfactory for one is unsatisfactory for another. Division of liquid samples is accomplished by means of a suitable division of the outflow of the tubing 19 (Fig. 1) into one or more replicates of chamber 21. Normally, a single detection device 27 serves to detect growth in all chambers 21. When the growth of potential varieties of micro-organisms requires provision of a different gas mixture in chamber 21 to allow growth of organisms that do not grow as well in usual conditions, separate detectors are used in order to avoid mixing the gas mixtures in separate chambers 21.

In the case of a catheterized patient, or in other applications, where it is desirable to detect the presence of any contamination whatsoever, the replaced growth medium should continue to be incubated with a gas detector after replacement for a sufficient length of time for any contamination of the last received sample to be cultured sufficiently to give off gas to be detected by the gas detector. In some applications, it is desirable to detect only the fact that the contamination of the sampled fluid has exceeded a certain minimum level, such as in a city drinking water supply or drinking water from fountains. By selecting the rate at which the growth medium is replaced to be sufficiently high and selecting the sensitivity of the radioactive gas detector to be sufficiently low, the radioactive gas detector will provide an indication of the existence of contamination only when the contamination of the sample being introduced into the growth medium exceeds a predetermined minimum level.

Thus, there is provided a system for maintaining substantially continuous monitoring of a fluid to detect incipient contamination thereof much more quickly than was possible with the prior art systems. The above description is of preferred embodiments of the invention and modifications may be made thereto without departing from the scope of the invention, which is defined in the appended claims.

## Claims

1. A system for automatically monitoring a fluid for contamination comprising a container adapted to contain a growth medium, and means for detecting changes that occur in association with microbial growth in the growth medium contained in said container, characterized by sampling means operable to take periodic samples of said fluid and periodically introduce said samples into the same growth medium in said container over an extended period of time, and said detecting means detecting changes that occur in association with microbial growth in said same growth medium during said extended period of time.

2. A system as recited in claim 1, wherein said sampling means controls the size of said sample to be small relative to the volume of the growth medium contained by said container so that a large number of successive uncontaminated samples can be introduced into said growth medium without substantially affecting the characteristics of said growth medium.

3. A system as recited in claim 1, wherein said fluid normally flows through tubing and wherein said sampling means periodically delivers into the growth medium contained by said container a sample of limited volume of the fluid flowing through said tubing.

4. A system as recited in claim 1, wherein said growth medium is a radio-labeled liquid growth medium and said detecting means is adapted to detect radio-active gases evolving from the growth medium.

5. A system as recited in claim 1, wherein said growth medium is a stable nuclide-labeled growth medium and said detecting means is adapted to detect stable nuclide-labeled gases evolving from said growth medium.

6. A system as recited in claim 1, wherein said sampling means comprises means to introduce a solid member into a stream of said fluid and then deliver said solid member into said growth medium contained by said container, said solid member being adapted to collect fluid from said stream by coming into contact with said fluid in said stream.

7. A system as recited in claim 6, wherein said solid member comprises a strip of material and said sampling means comprises means to incrementally and periodically advance said strip through said stream of fluid and then direct said strip into the growth medium contained in said container.

8. A system as recited in claim 1, wherein said sampling means comprises means to advance sample discs successively into a stream of said fluid and then transport the discs into the growth medium contained in said container, said sample discs comprising a material adapted to collect fluid from said stream by coming into contact with said stream.

9. A method of monitoring and detecting microbial contamination in a fluid comprising introducing a sample of said fluid into a growth medium and detecting changes that occur in the growth medium in association with microbial growth in the growth medium, characterized by taking samples of said fluid and periodically introducing said samples into the same growth medium over an extended period of time and detecting the changes that occur in association with microbial growth in this same growth medium over said extended period of time.

10. A method as recited in claim 9, wherein said growth medium is a radio-labeled growth medium.

11. A method as recited in claim 9, wherein said medium is a stable nuclide-labeled growth medium.

12. A method as recited in claim 9, wherein said fluid is a urine stream as it is discharged from a catheterized patient.

13. A method as recited in claim 9, wherein said periodic samples are taken at spaced time intervals.

14. A method as recited in claim 9, wherein said periodic samples are taken at least ten minutes apart.

15. A method as recited in any one of the claims 9 to 14, wherein the volume of said growth medium is at least ten times the volume of each of said samples so that a substantial number of uncontaminated samples can be successively introduced into said growth medium without substantially affecting the characteristics of said growth medium.

## Revendications

1. Système pour surveiller automatiquement un fluide en vue de déterminer sa contamination, comprenant un récipient destiné à contenir un milieu de croissance, et un dispositif pour déceler des changements se produisant en liaison avec une croissance microbienne dans le milieu de croissance contenu dans ce récipient, caractérisé par un dispositif d'échantillonnage pouvant agir pour prélever des échantillons périodiques du fluide ci-dessus et les introduire dans le milieu de croissance susdit se trouvant dans le récipient sur une période prolongée de temps, le dispositif de détection décelant des changements se produisant en liaison avec une croissance microbienne dans le milieu de croissance durant cette période prolongée de temps.

2. Système suivant la revendication 1, dans lequel le dispositif d'échantillonnage règle le volume de l'échantillon pour qu'il soit petit par rapport au volume du milieu de croissance contenu dans le récipient, de manière qu'un grand nombre d'échantillons non contaminés successifs puissent être introduits dans le milieu de croissance sans affecter sensiblement les caractéristiques de ce dernier.

3. Système suivant la revendication 1, dans lequel le fluide susdit circule normalement à travers une canalisation et le dispositif d'échantillon débite périodiquement, dans le milieu de croissance contenu dans le récipient, un échantillon de volume limité du fluide circulant dans cette canalisation.

4. Sysème suivant la revendication 1, dans lequel le milieu de croissance est un milieu de croissance liquide, marqué par éléments radioactifs et le dispositif de détection est destiné à déceler les gaz radioactifs se dégageant du milieu de croissance.

5. Sysème suivant la revendication 1, dans lequel le milieu de croissance est un milieu de croissance stable, marqué par nuclides et le dispositif de détection est destiné à déceler les gaz stables, marqués par nuclides, se dégageant du milieu de croissance.

6. Système suivant la revendication 1, dans lequel le dispositif d'échantillonnage comprend des moyens pour introduire un élément solide dans un courant du fluide ci-dessus et pour débiter ensuite cet élément solide dans le milieu de croissance contenu dans le récipient, cet élément solide étant destiné à récolter du fluide au départ du courant susdit en entrant en contact avec ce fluide dans le courant.

7. Système suivant la revendication 6, dans lequel l'élément solide consiste en une bande de matière et le dispositif d'échantillonnage comprend des moyens pour faire avancer graduellement et périodiquement cette bande à travers le courant de fluide et pour diriger ensuite cette bande dans le milieu de croissance contenu dans le récipient.

8. Système suivant la revendication 1, dans lequel le dispositif d'échantillonnage comprend des moyens pour faire avancer des disques d'échantillon en succession dans un courant du fluide ci-dessus et pour transporter ensuite ces disques dans le milieu de croissance contenu dans le récipient, ces disques d'échantillon comprenant une matière capable de récolter du fluide depuis le courant susdit par son entrée en contact avec celui-ci.

9. Procédé pour surveiller et déceler une contamination microbienne dans un fluide, comprenant l'introduction d'un échantillon de ce fluide dans un milieu de croissance et la détection des changements se produisant dans ce milieu de croissance en liaison avec une croissance microbienne dans ce milieu, caractérisé en ce qu'on prélève des échantillons du fluide ci-dessus et qu'on les introduit périodiquement dans le milieu de croissance susdit sur une période prolongée de temps, et on décèle les changements se produisant en liaison avec une croissance microbienne dans ce milieu de croissance sur cette période prolongée de temps.

10. Procédé suivant la revendication 9, dans lequel le milieu de croissance est un milieu de croissance marqué par éléments radioactifs.

11. Procédé suivant la revendication 9, dans lequel le milieu est un milieu de croissance stable marqué par nuclides.

12. Procédé suivant la revendication 9, dans lequel le fluide est un courant d'urine déchargé d'un patient portant un catheter.

13. Procédé suivant la revendication 9, dans lequel les échantillons périodiques sont prélevés à des intervalles espacés de temps.

14. Procédé suivant la revendication 9, dans lequel les échantillons périodiques sont prélevés à au moins 10 minutes d'intervalle.

15. Procédé suivant l'une quelconque des revendications 9 à 14, dans lequel le volume du milieu de croissance est au moins 10 fois le volume de chacun des échantillons de sorte qu'un nombre important d'échantillons non contaminés peuvent être introduits successivement dans le milieu de croissance susdit sans affecter sensiblement les caractéristiques de ce dernier.

**Patentansprüche**

1. Vorrichtung zum automatischen Überwachen eines Fluids auf Verunreinigungen, enthaltend einen zur Aufnahme eines Nährmittels geeigneten Behälter und eine Einrichtung zum Erfassen von Veränderungen, die in Verbindung mit einem mikrobischen Wachstum in dem im Behälter enthaltenen Nährmittel auftreten, gekennzeichnet durch eine Abtasteinrichtung zur periodischen Entnahme von Proben des Fluids und zur periodischen Eingabe der Proben in das im Behälter befindliche selbe Nährmittel über eine ausgedehnte Zeitspanne, wobei die Erfassungseinrichtung Veränderungen feststellt, die in Verbindung mit einem mikrobischen Wachstum in diesem selben Nährmittel während dieser ausgedehnten Zeitspanne auftreten.

2. Vorrichtung nach Anspruch 1, bei der die Abtasteinrichtung die Größe der Probe so steuert, daß sie im Vergleich zum Volumen des im Behälter enthaltenen Nährmittels klein ist, so daß eine große Anzahl aufeinanderfolgender verunreinigungsfreier Proben in dieses Nährmittel eingegeben werden kann, ohne daß dabei die Eigenschaften des Nährmittels in einer beachtenswerten Weise beeinträchtigt werden.

3. Vorrichtung nach Anspruch 1, bei der das Fluid normalerweise durch eine Rohrleitung fließt und bei der die Abtasteinrichtung in das im Behälter befindliche Nährmittel eine ein begreztes Volumen aufweisende Probe des durch die Rohrleitung fließenden Fluids periodisch eingibt.

4. Vorrichtung nach Anspruch 1, bei der das Nährmittel ein radioaktiv versetztes flüssiges Nährmittel ist und die Erfassungseinrichtung so ausgebildet ist, daß sie aus dem Nährmittel ausgeschiedene radioaktive Gase feststellen kann.

5. Vorrichtung nach Anspruch 1, bei der das Nährmittel ein stabiles mit Nucliden versetztes Nährmittel ist und daß die Erfassungseinrichtung derart ausgebildet ist, daß sie aus dem Nährmittel ausgeschiedene stabile mit Nucliden versetzte Gase erfassen kann.

6. Vorrichtung nach Anspruch 1, bei der die Abtasteinrichtung eine Einrichtung zum Einführen eines festen Körpers in einen Strom des Fluids und dann zum Abgeben des festen Körpers in das im Behälter befindliche Nährmittel aufweist, wobei der feste Körper in der Lage ist, Fluid aus dem Strom aufzunehmen, wenn er mit dem in dem Strom befindlichen Fluid in Berührung kommt.

7. Vorrichtung nach Anspruch 6, bei der der feste Körper einen Materialstreifen umfaßt und die Abtasteinrichtung eine Einrichtung zum schrittweisen und periodischen Vorschieben dieses Streifens durch den Fluidstrom und dann zum Einführen des Streifens in das im Behälter befindliche Nährmittel aufweist.

8. Vorrichtung nach Anspruch 1, bei der die Abtasteinrichtung eine Einrichtung zum aufeinanderfolgenden Vorschieben von Probenscheiben in einen Strom aus dem Fluid und dann zum Weiterbewegen der Scheiben in das im Behälter befindliche Nährmittel aufweist, wobei die Probenscheiben ein Material enthalten, das in der Lage ist, Fluid aus dem Strom aufzunehmen, wenn es in Berührung mit dem Strom kommt.

9. Verfahren zum Überwachen und Erfassen mikrobischer Verunreinigungen in einem Fluid, bei dem eine Probe des Fluids in ein Nährmittel eingebracht und Veränderungen erfaßt werden, die in dem Nährmittel in Verbindung mit einem darin vorkommenden mikrobischen Wachstum auftreten, dadurch gekennzeichnet, daß dem Fluid Proben entnommen und diese Proben in dasselbe Nährmittel über eine ausgedehnte Zeitspanne periodisch eingebracht werden und daß die Veränderungen festgestellt werden, die in Verbindung mit einem mikrobischen Wachstum in diesem selben Nährmittel während der ausgedehnten Zeitspanne auftreten.

10. Verfahren nach Anspruch 9, bei dem das Nährmittel ein radioaktiv versetztes Nährmittel ist.

11. Verfahren nach Anspruch 9, bei dem das Nährmittel ein stabiles mit Nucliden versetztes Nährmittel ist.

12. Verfahren nach Anspruch 9, bei dem das Fluid ein von einem katheterisierten Patienten abgegebener Urinstrom ist.

13. Verfahren nach Anspruch 9, bei dem die periodische Probenentnahme in voneinander beabstandeten Zeitintervallen erfolgt.

14. Verfahren nach Anspruch 9, bei dem die periodische Probenentnahme wenigstens in Abständen von 10 min erfolgt.

15. Verfahren nach einem der Ansprüche 9 bis 14, bei dem das Volumen des Nährmittels wenigstens das Zehnfache des Volumens jeder der Proben ausmacht, so daß eine hinreichende Anzahl verunreinigungsfreier Proben aufeinanderfolgend in das Nährmittel eingebracht werden kann, ohne daß dabei die Eigenschaften des Nährmittels in einem beachtlichen Maße beeinträchtigt werden.

FIG. 1.

FIG. 2.

*FIG. 3.*

IONIZATION DETECTOR — 45

43

37

41

27

*FIG. 4.*

GEIGER-MUELLER COUNTER — 46

THIN WALL

48

47

27

*FIG. 7.*

BYPASS MODE

13

51

19

FILTER VENT

61

59

17

SOLENOID — 53

57

TIMER

*FIG. 8.*

SAMPLE MODE

13

51

19

FILTER VENT

61

59

17

SOLENOID — 53

57

TIMER

*FIG. 9.*

13

83

80

79

77

71

19

17

73

75

85

TIMER

*FIG. 10.*

83

80

79

77

71

75

73

85

TIMER

2

FIG. 5.

SEMICONDUCTOR
DETECTOR
49

47

FIG. 6.

NUCLEAR
MAGNETIC
RESONANCE
ANALYZER
50

47

101

FIG. 11.

99

103

FIG. 12.

100 100

FLUID FLOW
IN

FLUID FLOW
OUT

STEPPING
MOTOR

TIMER

99

115

117

103

3

0 037 416

FIG. 13.

FIG. 14.

FIG. 15.

FIG. 16.

FIG. 17.

FIG. 18.

FIG. 19.

D1
D2
113
100
112
103
111

D1
D2
D3
111
112
113
103

D2
D3
D4
112
111
113
D1
TO GROWTH
MEDIUM CHAMBER

120
122

141
133
131
135
FLUID FLOW IN
FLUID FLOW OUT
139
137

131
143
145

131
143
145

4